# EUROPEAN PATENT APPLICATION

(11) **EP 3 323 408 A1**
(43) Date of publication of application: **23.05.2018**
(21) Application number: 16200085.5
(22) Date of filing: 22.11.2016
(51) Int. Cl.: A61K 8/365, A61Q 5/06

(54) **METHOD OF SHAPING HAIR**

(71) Applicant: Unilever PLC, London Greater London EC4Y 0DY (GB)
(72) Inventor: MURRAY, Andrew Malcolm, Neston, Cheshire, CH64 6XG (GB); PAUL, Prem Kumar Cheyalazhagan, Wirral, Merseyside, CH63 3JW (GB); ROBINSON, Teresa Maria, Wirral, Merseyside, CH63 3JW (GB)
(74) Representative: James, Helen Sarah

(57) **Abstract**

The invention provides a method for shaping hair which comprises subjecting the hair to a series of consecutive treatments, wherein each treatment comprises the following steps:
(i) topically applying a hair shaping composition to the hair;
(ii) allowing the composition to remain on the hair for a period of from 10 to 180 seconds;
(iii) rinsing the treated hair, and
(iv) mechanically shaping the rinsed hair by contacting it with a heated hair shaping device at a temperature of at least 50°C;
and wherein the number of consecutive treatments in the series is from 2 to 10;
and wherein the time interval between each two consecutive treatments in the series is from 8 hours to 30 days;
and wherein the hair shaping composition has a pH of 4 or less and comprises from 0.05 to 1.5% (by weight based on the total weight of the composition) of glyoxylic acid or a salt or hydrate thereof.

## Description

### Field of the Invention

This invention relates to a hair shaping composition, and more particularly a hair shaping composition which does not require the breakage of hair disulfide bonds.

### Background and Prior Art

Hair straightening or relaxing has become increasingly popular in view of hair styles which require relatively or perfectly straight hair.

Hair straightening or relaxer products most commonly used in salons and in the home contain as the active hair-straightening agent either a strong alkali, such as sodium hydroxide or guanidine hydroxide; a sulfite, typically an ammoniacal mixture of bisulfite and sulfite; or a thiol compound, typically ammonium thioglycolate.

All of the above types of product exert their primary effect by breaking the natural cystine disulfide bonds present in hair keratin. Repeated treatments over time may leave the hair weaker and more prone to breakage. Some straightening or relaxer products may also have an unpleasant odour and irritate the scalp.

In response to the above concerns, efforts have been made to develop treatments which can provide long-lasting hair straightness benefits without involving breakage of the hair disulfide bonds. However, the application methods prescribed are generally of the "one-shot" type. Compared to the normal protocols used by consumers to style their hair, "one-shot" treatments typically use much higher levels of product, a longer contact time of product on hair, and an increased number of passes of heated hair shaping tools such as hair straightening irons per individual treatment. This is not always convenient or desirable for the consumer.

The present invention addresses this problem.

### Summary of the Invention

The present invention provides a method for shaping hair which comprises subjecting the hair to a series of consecutive treatments, wherein each treatment comprises the following steps:
(i) topically applying a hair shaping composition to the hair;
(ii) allowing the composition to remain on the hair for a period of from 10 to 180 seconds;
(iii) rinsing the treated hair, and
(iv) mechanically shaping the rinsed hair by contacting it with a heated hair shaping device at a temperature of at least 50°C;
and wherein the number of consecutive treatments in the series is from 2 to 10;
and wherein the time interval between each two consecutive treatments in the series is from 8 hours to 30 days;
and wherein the hair shaping composition has a pH of 4 or less and comprises from 0.05 to 1.5% (by weight based on the total weight of the composition) of glyoxylic acid or a salt or hydrate thereof.

### Detailed Description and Preferred Embodiments

All molecular weights as used herein are weight average molecular weights, unless otherwise specified.

A hair shaping composition for use in the invention will generally have an aqueous continuous phase, i.e. a continuous phase which has water as its basis. Typically, such a composition will comprise from 50 to 99.5% water, preferably from about 80 to about 99.5% water (by weight based on the total weight of the composition). Other organic solvents may also be present, such as lower alkyl alcohols and polyhydric alcohols. Examples of lower alkyl alcohols include C₁ to C₆ monohydric alcohols such as ethanol and isopropanol. Examples of polyhydric alcohols include propylene glycol, hexylene glycol, glycerin, and propanediol. Mixtures of any of the above-described organic solvents may also be used.

The hair shaping composition for use in the invention comprises from 0.05 to 1.5% (by weight based on the total weight of the composition) of glyoxylic acid or a salt or hydrate thereof.

Glyoxylic acid (OHC-COOH) is known to exist as a hydrate of formula (I) under certain conditions. 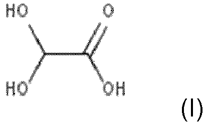 Also, glyoxylic acid may exist as a mixture of both the aldehyde and hydrate forms. The hydrate may also condense to dimers of formula (II).

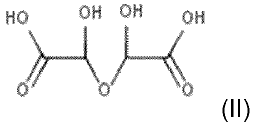

Glyoxylic acid also forms salts with counterions such as alkali metal (preferably sodium), alkaline-earth metal (preferably calcium), ammonium and substituted ammonium ions.

Any of these forms or a mixture of any of these forms are suitable for use in the invention.

Preferably glyoxylic acid in aqueous solution is used in the composition for use in the invention. Glyoxylic acid in aqueous solution generally exists as the hydrate of formula (I) together with a small proportion of the dimer of formula (II).

Preferably, the level of glyoxylic acid or salt or hydrate thereof ranges from 0.1 to 1.2%, more preferably from 0.2 to 1% and most preferably from 0.5 to 0.9% by weight based on the total weight of the composition.

A particularly preferred composition for use in the invention comprises glyoxylic acid hydrate of formula (I), in an amount ranging from 0.1 to 1.2%, more preferably from 0.2 to 1% and most preferably from 0.5 to 0.9% by weight based on the total weight of the composition.

A hair shaping composition for use in the invention may preferably include additional active ingredients for the shaping of hair. Examples of such ingredients include polyhydroxy acid lactones, aliphatic di- or tricarboxylic acids or salts or hydrates thereof; and mixtures thereof.

Polyhydroxy acid lactones for use in the invention are typically aliphatic compounds having at least two hydroxyl groups in the molecule and with a preferred molecular weight of between 100 and 300.

Preferred polyhydroxy acid lactones for use in the invention contain from 3 to 8 carbon atoms and from 2 to 8 hydroxyl groups, with at least two of the hydroxyl groups being respectively attached to two adjacent carbon atoms of the molecule.

Illustrative examples of such materials include:

### (a) Aldonic acid lactones

Aldonic acids are polyhydroxy acids resulting from oxidation of the aldehyde group of an aldose to a carboxylic acid group, and the acid of which can be represented by the following general formula:

H(CHOH)ₙCH(OH)COOH

where n is an integer from 1 to 6.

The aldonic acids can exist as stereoisomers as D, L and DL or R, S and RS forms. The aldonic acids form intramolecular lactones by removing one mole of water between the carboxyl group and one hydroxyl group.

The following are representative aldonic acid lactones:
2,3-dihydroxypropanoic acid lactones (glyceric acid lactone);
2,3,4-trihydroxybutanoic acid lactones (stereoisomers: erythronolactone, threonolactone);
2,3,4,5-tetrahydroxypentanoic acid lactones (stereoisomers: ribonolactone, arabinolactone, xylonolactone, lyxonolactone);
2,3,4,5,6-pentahydroxyhexanoic acid lactones (stereoisomers: allonolactone, altronolactone, gluconolactone, mannolactone, gulonolactone, idonolactone, galactonolactone, talonolactone), and
2,3,4,5,6,7-hexahydroxyheptanoic acid lactones (stereoisomers: alloheptonolactone, altroheptonolactone, glucoheptonolactone, mannoheptonolactone, guloheptonolactone, idoheptonolactone, galactoheptonolactone, taloheptonolactone).

### (b) Aldaric acid lactones

Aldaric acids are polyhydroxy dicarboxylic acids derived from an aldose by oxidation of both terminal carbon atoms to carboxyl groups, and the acid of which can be represented by the following general formula:

HOOC(CHOH)ₙCH(OH)COOH

where n is an integer from 1 to 4.

The aldaric acids can exist as stereoisomers as D, L and DL or R, S and RS forms. The aldaric acids form intramolecular lactones by removing one mole of water between one carboxyl group and one hydroxyl group.

The following are representative aldaric acid lactones:
2,3-dihydroxybutane-1,4-dioic acid lactones
2,3,4-trihydroxypentane-1,5-dioic acid lactones (stereoisomers: ribarolactone, arabarolactone, xylarolactone, lyxarolactone);
2,3,4,5-tetrahydroxyhexane-1,6-dioic acid lactones (allarolactone, altrarolactone, glucarolactone, mannarolactone, gularic acid and gularolactone, idarolactone, galactarolactone, talarolactone);
2,3,4,5,6-pentahydroxyheptane-1,7-dioic acid lactones (stereoisomers: alloheptarolactone, altroheptarolactone, glucoheptarolactone, mannoheptarolactone, guloheptarolactone, idoheptarolactone, galactoheptarolactone, taloheptarolactone).

### (c) Alduronic acids

Alduronic acids are polyhydroxy acids resulting from oxidation of the alcohol group of an aldose to a carboxylic acid group, and can be represented by the following general formula:

HOOC(CHOH)ₙCH(OH)CHO

where n is an integer from 1 to 4.

The alduronic acids can exist as stereoisomers as D, L and DL or R, S and RS forms. The alduronic acids form intramolecular lactones by removing one mole of water between the carboxyl group and one hydroxyl group.

The following are representative alduronic acid lactones:
riburonolactone; araburonolactone; xyluronolactone; lyxuronolactone; alluronolactone; altruronolactone; glucuronolactone; mannuronolactone; guluronolactone; iduronolactone; galacturonolactone; taluronolactone; allohepturonolactone; altrohepturonolactone; glucohepturonolactone; mannohepturonolactone; gulohepturonolactone; idohepturonolactone; galactohepturonolactone and talohepturonolactone.

Particularly preferred polyhydroxy acid lactones for use in the invention may be selected from gluconolactone, galactonolactone, glucuronolactone, galacturonolactone, gulonolactone, ribonolactone, saccharic acid lactone, pantoyllactone, glucoheptonolactone, mannonolactone, galactoheptonolactone, and mixtures thereof. Most preferred polyhydroxy acid lactones for use in the invention may be selected from D-glucono-δ-lactone, D-glucono-γ-lactone, D-gluconic acid and mixtures thereof.

D-glucono-δ-lactone is the cyclic 1,5-intramolecular ester of D-gluconic acid, which is produced industrially by enzymatic oxidation of D-glucose.

D-glucono-δ-lactone *per se* is available in the form of a particulate solid (e.g. a crystalline powder). In aqueous media it may exist in an equilibrium mixture together with its isomer (D-glucono-γ-lactone), and its hydrolysis product (D-gluconic acid). Any of these forms or a mixture of any of these forms are suitable for use in the invention. Most preferably D-glucono-δ-lactone *per se* is used.

When included in a composition for use in the invention, the level of polyhydroxy acid lactone preferably ranges from 0.5 to 6%, more preferably from 1 to 3% and most preferably from 1.5 to 2.5% by weight based on the total weight of the composition.

A particularly preferred composition for use in the invention comprises D-glucono-δ-lactone, in an amount ranging from 0.5 to 6%, more preferably from 1 to 3% and most preferably from 1.5 to 2.5% by weight based on the total weight of the composition.

Aliphatic di- or tricarboxylic acids for use in the invention typically have a molecular weight (M_{W}) ranging from 60 to 300 g/mol and at least one pKₐ value (measured at 25°C in water) ranging from 2.5 to 4.

Illustrative examples of dicarboxylic acids for use in the invention correspond to the following general formula:

HOOC-R¹-COOH

in which R¹ is a divalent, saturated or unsaturated, linear or branched hydrocarbyl radical having from 1 to 4 carbon atoms, and which may optionally be substituted with one or more hydroxyl groups.

Preferably R¹ is a divalent saturated linear alkyl radical of formula -[CH(X)]ₙ- in which n is an integer ranging from 1 to 3 and each X is independently selected from -H and - OH.

Specific examples of such dicarboxylic acids include malonic acid and tartaric acid.

Illustrative examples of tricarboxylic acids for use in the invention correspond to the following general formula:

HOOC-CH₂-R²-COOH

in which R² is a divalent, saturated or unsaturated, linear or branched hydrocarbyl radical having from 1 to 3 carbon atoms, which is substituted with one -COOH group and which may optionally be substituted with one or more hydroxyl groups.

Specific examples of such tricarboxylic acids include citric acid, aconitic acid and tricarballylic acid.

Suitable salts include those with counterions such as alkali metal (preferably sodium), alkaline-earth metal (preferably calcium), ammonium and substituted ammonium ions.

Mixtures of any of the above-described aliphatic di- or tricarboxylic acids or salts or hydrates thereof may also be used.

Particularly preferred aliphatic di- or tricarboxylic acids for use in the invention may be selected from citric acid or salts or hydrates thereof, such as monosodium citrate, trisodium citrate, tricalcium citrate, trisodium citrate dihydrate, tripotassium citrate, monosodium citrate anhydrous, citric acid anhydrous, citric acid monohydrate and mixtures thereof.

Most preferred aliphatic di- or tricarboxylic acids for use in the invention may be selected from citric acid anhydrous and/or citric acid monohydrate.

When included in a composition for use in the invention, the level of aliphatic di- or tricarboxylic acid preferably ranges from 0.5 to 6%, more preferably from 1 to 3% and most preferably from 1.5 to 2.5% by weight based on the total weight of the composition.

A particularly preferred composition for use in the invention comprises citric acid anhydrous and/or citric acid monohydrate, in an amount ranging from 0.5 to 6%, more preferably from 1 to 3% and most preferably from 1.5 to 2.5% by weight based on the total weight of the composition.

A most preferred composition for use in the invention comprises D-glucono-δ-lactone, in an amount ranging from 1.5 to 2.5% by weight based on the total weight of the composition; and citric acid anhydrous and/or citric acid monohydrate, in an amount ranging from 1.5 to 2.5% by weight based on the total weight of the composition.

Advantageously, the hair shaping composition for use in the invention does not require the incorporation of reducing agents, and a hair shaping composition for use in the invention is generally substantially free of such materials.

The term "substantially free" in the context of this invention means that reducing agents are absent or included in trace quantities only, such as no more than 0.1 %, preferably no more than 0.01%, and more preferably from 0 to 0.001% by weight based on the total weight of the composition.

The term "reducing agent" in the context of this invention means an agent which is effective to break hair disulfide bonds when applied to hair for a period ranging from 3 to 15 minutes and at a temperature ranging from 20 to 30°C. Examples of such reducing agents are ammonium thioglycolate (in a solution having a pH of between 7 and 10.5), glyceryl monothioglycolate (employed at a pH of less than 7), thioglycolic acid, dithioglycolic acid, mercaptoethyl amine, mercaptopropionic acid, dithioglycolate and alkali metal or ammonium sulfites or bisulfites.

A hair shaping composition for use in the invention may suitably include a structured liquid phase, which may be characterized as a lamellar gel network formed from cationic surfactant and fatty alcohol bilayers. The bilayers may grow, swell or fold to form extended sheets or spherical vesicles in the aqueous continuous phase of the composition.

Examples of suitable cationic surfactants include cetyltrimethylammonium chloride (CTAC), behenyltrimethylammonium chloride (BTAC) and mixtures thereof.

The level of cationic surfactant suitably ranges from 0.1 to 10%, preferably from 0.2 to 5% and more preferably from 0.25 to 4% (by weight based on the total weight of the composition).

Examples of suitable fatty alcohols include cetyl alcohol, stearyl alcohol, behenyl alcohol, and mixtures thereof.

The level of fatty alcohol suitably ranges from 0.01 to 10%, preferably from 0.1 to 8%, more preferably from 0.2 to 7% and most preferably from 0.3 to 6% (by weight based on the total weight of the composition).

The weight ratio of cationic surfactant to fatty alcohol is suitably from 1:1 to 1:10, preferably from 1:1.5 to 1:8, optimally from 1:2 to 1:5.

A hair shaping composition for use in the invention may also incorporate other optional ingredients to enhance performance and/or consumer acceptability. Suitable optional ingredients include: preservatives, colouring agents, chelating agents, antioxidants, fragrances, antimicrobials, antidandruff agents, cationic conditioning polymers, ingredients, sunscreens, proteins and hydrolysed proteins.

The pH of the hair shaping composition for use in the invention is 4 or less, and preferably ranges from 1.5 to 3.8, more preferably from 2.5 to 3.5 and most preferably from 2.8 to 3.2.

The hair shaping composition for use in the invention is suitable for topical application to hair for improved hair volume-down. The term "volume-down" in the context of this invention generally means reduced visible bulkiness of the hair. For many consumers, improved hair volume-down provides a number of associated benefits, such as improved straightness, smoothness, manageability and style retention.

In the method according to the invention, the hair shaping composition (as is further described above) is topically applied to the hair in a series of consecutive treatments. Each treatment comprises the following steps:
(i) topically applying the hair shaping composition to the hair;
(ii) allowing the composition to remain on the hair for a period of from 10 to 180 seconds;
(iii) rinsing the treated hair, and
(iv) mechanically shaping the rinsed hair by contacting it with a heated hair shaping device at a temperature of at least 50°C.

In step (i) of the above treatment, generally any application amount of hair shaping composition that covers the hair to be treated suffices. In studies on hair tresses, an application level of from 0.05 to 0.5 ml, preferably 0.1 to 0.3 ml of hair shaping composition per gram of hair was found to yield effective results, corresponding to a an application level of about 0.0004 to 0.004g, preferably 0.0008 to 0.0024g of glyoxylic acid or salt or hydrate thereof per gram of hair. Lesser amounts may be used, for example, if only a section of hair, just the hair tips, or hair roots are to be treated. If the hair has been treated with an excess of the hair shaping composition, the hair may be lightly patted with a towel to absorb any surplus hair shaping composition, or the surplus may be removed with the fingers.

The consumer may prefer that the hair be shampooed and/or towel dried before step (i). It may also be preferred that the hair is damp, as it may aid in the distribution of the hair shaping composition, but dry hair may also be treated. The hair shaping composition is preferably uniformly delivered, for example by working it from the root end to the tip end of the hair.

In step (ii) of the above treatment, it is preferred that the hair shaping composition is allowed to remain on the hair for a period of from 30 to 90 seconds, more preferably from 45 to 75 seconds and most preferably from 55 to 65 seconds.

In step (iii) of the above treatment, the treated hair is preferably rinsed with water for a period of from 5 to 30 seconds, more preferably from 10 to 20 seconds.

Preferably the rinsed hair is dried prior to commencement of mechanical shaping step (iv).The hair thus treated may be dried naturally by exposure to air, by use of a heated hair drying appliance, by rubbing with a water-absorbent article, or by a combination of any of these methods.

In step (iv) the rinsed (and preferably dried) hair is mechanically shaped by contacting it with a heated hair shaping device at a temperature of at least 50° C.

The term "at a temperature of at least 50° C" in the context of this invention generally means that the surface temperature of the portion of the device that contacts the hair is at least 50° C. Preferably the temperature ranges from 100°C to 230° C, more preferably from 120°C to about 220° C and most preferably from 170° C to 200° C.

A variety of heated devices are available for shaping hair. Many of these devices function by incorporating an internal or external heating source for heating a surface of the device that contacts the hair. The application of heat drives out residual water from the hair and encourages the formation of more bonds between hair proteins, helping to set the hair in the desired shape.

The hair may for example be shaped using a heated hairdryer with an attachment. Such attachments include brushes, combs or diffusers that attach to the heated end of the hairdryer nozzle and allow for heated hair shaping.

Alternatively heated hair shaping tools such as hair straighteners and hair curling irons can be used separately from a hairdryer. Such tools heat up and the heated portion can be used to shape the hair. Typically the heated portion incorporates a shaped surface for contacting the hair so as to manipulate the hair to conform to the shape of the surface. When using a shaped surface, a flat surface may be used for straightening hair and a curved surface may be used for curling the hair, or a combination of such surfaces may be used, if desired. Hair straighteners function by compressing hair between flat, heated surfaces that "iron" the hair. Similar devices can be used for "crimping" or creating waves to hair surfaces. Curling irons include an elongated tubular barrel which includes a heating element generally located inside the barrel. The unheated clamp extends along the heated barrel and clamps down to hold portions of a user's hair against the heated barrel. Heat from the heated barrel causes the hair on the barrel to retain its shape and curl after loosening the clamp. Also available are heated curling brushes that act in a manner similar to a curling iron, but include elongated teeth extending from the barrel which brush or combs the hair while curling without the use of a clamp. Heated curlers or rollers may also be used to shape hair.

Preferably the heated hair shaping device for use in step (iv) is a hair straightener, and more preferably a hair straightening iron which compresses hair between flat heated surfaces at a temperature ranging from 100°C to 230° C, more preferably from 120°C to about 220° C and most preferably from 170° C to 200° C. Such a hair straightening iron may be applied to hair by successive separate touches of a few seconds (e.g. from 2 to 10 seconds per touch), or by gradually moving or sliding it along the hair. Preferably the hair straightening iron is drawn along the length of the hair in a continuous pass from the root end to the tip end. Repeated passes of the hair straightening iron may be carried out along the same length of hair, with each pass generally lasting from about 5 seconds to 1 minute and with intermediate removal of the flat heated surfaces from the hair between passes. For the purposes of the present invention, the number of passes of the hair straightening iron along the same length of hair is suitably no more than 4, and preferably ranges from 1 to 4, more preferably from 1 to 3 and most preferably from 2 to 3.

In the method according to the invention, steps (i) to (iv) as described above are repeated in a series of consecutive treatments, and the number of consecutive treatments in the series is from 2 to 10. Preferably the number of consecutive treatments ranges from 3 to 7, more preferably from 4 to 6.

The time interval between each two consecutive treatments in the series is from 8 hours to 30 days. Preferably the time interval ranges from 12 hours to 1 week, more preferably from 18 hours to 3 days and most preferably from 20 to 30 hours, such as daily.

Surprisingly, the inventors have found that the method according to the invention is capable of providing durable shaping to hair. The term "durable shaping" in the context of this invention generally means shaping which is capable of persisting after being subjected to one or more washes, preferably at least two washes and more preferably at least three washes, such as four or five washes.

The invention is further illustrated with reference to the following, non-limiting Examples, in which all percentages are by weight based on total weight unless otherwise specified.

### EXAMPLES

### Examples 1 and 2 and (Comparative) Example A

Dark brown European wavy#6 switches of length 25 cm and weight 2gms, were washed with shampoo. The following test solutions were prepared:
Example 1: Aqueous solution, 0.8% glyoxylic acid
Example A: Aqueous solution, 2% citric acid and 2% D-glucono-δ-lactone
Example 2: Aqueous solution, 0.8% glyoxylic acid, 2% citric acid and 2% D-glucono-δ-lactone

0.4ml of each of the respective test solutions was applied to the damp switches (5 switches for each solution) and left on for 1 minute. The switches were then rinsed for 15 seconds prior to blow drying and ironing with 3 passes using FHI straightening irons at 200°C. 5 switches with no product on were blow dried and ironed and used as control switches with which to compare.

The switches after ironing were left overnight under ambient conditions before they were washed using shampoo, combed straight and left to dry. Once dry the switches were combed straight and the images of the switches captured. If the switches are visually straight, then the volume of the switches gives the straightening benefit of the actives. (Here volume refers to the projection of the switch image on to the screen and is given in mm²). This was repeated another 4 times and the results are shown in Table 1.

**Table 1**

| Volumes of treated hair switches in mm² after a single heat treatment and subsequent three washes. | | | | |
|---|---|---|---|---|
| **Treatment** | **Volume in mm²** | | | **%benefit between 5th and 1st treatment** |
| | **Treatment 1** | **Treatment 3** | **Treatment 5** | |
| Example 1 | 15269 | 13649 | 12393 | 18.8 |
| Example A | 14267 | 13030 | 13411 | 6.0 |
| Example 2 | 17295 | 13441 | 11154 | 35.5 |
| Control (water) | 18440 | 17229 | 17604 | 4.5 |

From the table it can be seen that the switches treated with Examples 1 and 2 (according to the method of the invention) show excellent progressive benefits over the 5 treatment cycles. The combination of Example 2 provides greatly enhanced progressive benefits in both relative and absolute terms, which is indicative of a synergistic effect.

In a "wash out" experiment, the switches treated as described above with the test solutions of Examples 1 and 2 were washed 5 times with no intervening treatments applied or straightening done in between washes. The switches showed excellent retention of straightness benefits even after 5 washes.

The results show that the method of the invention provides progressive straightness benefits to hair after 5 treatments; and that these benefits last for at least 5 washes following the 5^{th} treatment.

### Example 3

The following formulation illustrates a hair shaping composition for use according to the invention

| **Ingredient** | **% activity** | **%w/w raw material** |
|---|---|---|
| Behentrimonium chloride | 70 | 2.0 |
| Cetearyl alcohol | 100 | 4.0 |
| Glyoxylic Acid | 100 | 0.8 |
| D-glucono-δ-lactone | 100 | 2.0 |
| Citric acid | 100 | 2.0 |
| Water | 100 | To 100% |
| pH = 3.0 | | |

## Claims

1. A method for shaping hair which comprises subjecting the hair to a series of consecutive treatments, wherein each treatment comprises the following steps:
(i) topically applying a hair shaping composition to the hair;
(ii) allowing the composition to remain on the hair for a period of from 10 to 180 seconds;
(iii) rinsing the treated hair, and
(iv) mechanically shaping the rinsed hair by contacting it with a heated hair shaping device at a temperature of at least 50°C;
and wherein the number of consecutive treatments in the series is from 2 to 10; and wherein the time interval between each two consecutive treatments in the series is from 8 hours to 30 days;
and wherein the hair shaping composition has a pH of 4 or less and comprises from 0.05 to 1.5% (by weight based on the total weight of the composition) of glyoxylic acid or a salt or hydrate thereof.

2. A method according to claim 1, in which the hair shaping composition comprises glyoxylic acid hydrate of formula (I), 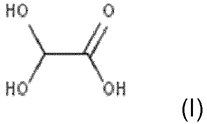 in an amount ranging from 0.5 to 0.9% by weight based on the total weight of the composition.

3. A method according to claim 1 or claim 2, in which the hair shaping composition includes additional active ingredients for the shaping of hair which are selected from polyhydroxy acid lactones, aliphatic di- or tricarboxylic acids or salts or hydrates thereof; and mixtures thereof.

4. A method according to claim 3, in which the hair shaping composition comprises D-glucono-δ-lactone, in an amount ranging from 1.5 to 2.5% by weight based on the total weight of the composition; and citric acid anhydrous and/or citric acid monohydrate, in an amount ranging from 1.5 to 2.5% by weight based on the total weight of the composition.

5. A method according to any one of claims 1 to 4, in which in step (i) the application level of glyoxylic acid or salt or hydrate thereof is from 0.0008 to 0.0024g per gram of hair.

6. A method according to any one of claims 1 to 5, in which in step (ii) the hair shaping composition is allowed to remain on the hair for a period of from 55 to 65 seconds.

7. A method according to any one of claims 1 to 6, in which in step (iv) the heated hair shaping device is a hair straightening iron which compresses hair between flat heated surfaces at a temperature ranging from 100°C to 230° C.

8. A method according to claim 7, in which the hair straightening iron is drawn along the length of the hair in a continuous pass from the root end to the tip end, and the number of passes of the hair straightening iron along the same length of hair ranges from 1 to 3, with each pass lasting from 5 seconds to 1 minute and with intermediate removal of the flat heated surfaces from the hair between passes.

9. A method according to any one of claims 1 to 8, in which the number of consecutive treatments in the series is from 4 to 6.

10. A method according to any one of claims 1 to 9, in which the time interval between each two consecutive treatments in the series is from 18 hours to 3 days.
